(19) Europäisches Patentamt
European Patent Office
Office européen des brevets

(11) **EP 2 831 263 B1**

(12) **EUROPEAN PATENT SPECIFICATION**

(45) Date of publication and mention of the grant of the patent:
**28.06.2017 Bulletin 2017/26**

(21) Application number: **13712795.7**

(22) Date of filing: **27.03.2013**

(51) Int Cl.:
***C12Q 1/00*** *(2006.01)*

(86) International application number:
**PCT/EP2013/056619**

(87) International publication number:
**WO 2013/144255 (03.10.2013 Gazette 2013/40)**

(54) **IMPROVED SPACER MEMBRANE FOR AN ENZYMATIC IN-VIVO SENSOR**

VERBESSERTE ABSTANDSHALTERMEMBRAN FÜR EINEN ENZYMATISCHEN IN-VIVO-SENSOR

MEMBRANE D'ESPACEMENT AMÉLIORÉ POUR UN CAPTEUR ENZYMATIQUE IN VIVO

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2012 PCT/EP2012/055406**

(43) Date of publication of application:
**04.02.2015 Bulletin 2015/06**

(60) Divisional application:
**17169444.1**

(73) Proprietors:
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**
- **Roche Diabetes Care GmbH**
  **68305 Mannheim (DE)**
  Designated Contracting States:
  **DE**

(72) Inventors:
- **STAIB, Arnulf**
  **64646 Heppenheim (DE)**
- **THIELE, Marcel**
  **68309 Mannheim (DE)**
- **KOELKER, Karl-Heinz**
  **67269 Grünstadt (DE)**
- **RIEGER, Ewald**
  **67240 Bobenheim-Roxheim (DE)**
- **Licht, Alexander**
  **69469 Weinheim (DE)**

(74) Representative: **Weickmann & Weickmann PartmbB**
**Postfach 860 820**
**81635 München (DE)**

(56) References cited:
**EP-A1- 2 163 190**

- **MANG A ET AL: "Biocompatibility of an electrochemical sensor for continuous glucose monitoring in subcutaneous tissue", DIABETES TECHNOLOGY AND THERAPEUTICS, vol. 7, no. 1, 1 February 2005 (2005-02-01), pages 163-173, XP002446481, MARY ANN LIEBERT, LARCHMONT, NY, US ISSN: 1520-9156, DOI: 10.1089/DIA.2005.7.163**
- **UCHIYAMA T ET AL: "Biocompatible polymer alloy membrane for implantable artificial pancreas", JOURNAL OF MEMBRANE SCIENCE, vol. 208, no. 1-2, 1 October 2002 (2002-10-01), pages 39-48, XP004380080, ELSEVIER SCIENTIFIC PUBL.COMPANY. AMSTERDAM, NL ISSN: 0376-7388, DOI: 10.1016/S0376-7388(02)00137-0**
- **YAN WANG ET AL: "Polymeric "smart" coatings to prevent foreign body response to implantable biosensors", JOURNAL OF CONTROLLED RELEASE, 5 January 2013 (2013-01-05), XP055061963, ISSN: 0168-3659, DOI: 10.1016/j.jconrel.2012.12.028**
- **JACQUELINE M. MORAIS ET AL: "Biomaterials/Tissue Interactions: Possible Solutions to Overcome Foreign Body Response", THE AAPS JOURNAL, vol. 12, no. 2, June 2010 (2010-06), pages 188-196, XP055061980, ISSN: 1550-7416, DOI: 10.1208/s12248-010-9175-3**

EP 2 831 263 B1

**(Cont. next page)**

- Anonymous: "Adapt TM Biostable Drug Delivery Platform", BioInteractions , 5 January 2012 (2012-01-05), page 1, XP002696778, Retrieved from the Internet: URL:http://www.biointeractions.com/pdfs/adapt_overview.pdf [retrieved on 2013-05-07]
- ALI OZGUR BOZTAS ET AL: "Immobilization and Release of the Redox Mediator Ferrocene Monocarboxylic Acid from within Cross-Linked p(HEMA- co -PEGMA- co -HMMA) Hydrogels", BIOMACROMOLECULES, vol. 10, no. 8, 10 August 2009 (2009-08-10), pages 2135-2143, XP055062479, ISSN: 1525-7797, DOI: 10.1021/bm900299b
- ABRAHAM S ET AL: "Molecularly engineered p(HEMA)-based hydrogels for implant biochip biocompatibility", BIOMATERIALS, vol. 26, no. 23, 1 August 2005 (2005-08-01), pages 4767-4778, XP027768280, ELSEVIER SCIENCE PUBLISHERS BV., BARKING, GB ISSN: 0142-9612 [retrieved on 2005-08-01]

**Description**

The present invention relates to an electrode system for measuring the concentration of an analyte under *in-vivo* conditions, comprising an electrode with immobilized enzyme molecules and an improved diffusion barrier that controls diffusion of the analyte from body fluid surrounding the electrode system to the enzyme molecules.

Further, the present invention relates to an electrode system for measuring the concentration of an analyte under *in-vivo* conditions, comprising an electrode with immobilized enzyme molecules, optionally a diffusion barrier that controls diffusion of the analyte from the exterior of the electrode system to the enzyme molecule and an improved spacer membrane which forms at least a portion of the outer layer of the electrode system.

Sensors with implantable or insertable electrode systems facilitate measurements of physiologically significant analytes such as, for example, lactate or glucose in a patient's body. The working electrodes of systems of this type have electrically conductive enzyme layers in which enzyme molecules are bound which release charge carriers by catalytic conversion of analyte molecules. In the process, an electrical current is generated as measuring signal whose amplitude correlates to the analyte concentration.

Such electrode systems are e.g. known from WO 2007/147475 and WO 2010/028708.

The working electrodes of the electrode system are provided with a diffusion barrier that controls the diffusion of the analyte to be determined from the body fluid or tissue surrounding the electrode system to the enzyme molecules that are immobilized in the enzyme layer. According to WO 2010/028708, the diffusion barrier of the electrode system is a solid solution of at least two different polymers, preferably of acrylates. The polymers may be copolymers, e.g. copolymers of methyl methacrylate and hydroxyethyl methacrylate or copolymers of butyl methacrylate and hydroxyethyl methacrylate.

WO 2007/147475 discloses a diffusion barrier made from a polymer having a zwitterionic structure. An example of such a polymer is poly(2-methacryloyloxyethyl phosphorylcholine-co-n-butylmethacrylate). The zwitterionic polymer may be mixed with another polymer, for example polyurethane.

The use of polymer or copolymer mixtures, however, has drawbacks in that the preparation of the mixture and its application to the sensor is tedious and potentially problematic. Usually, the polymers to be mixed are individually dissolved and the resulting solutions are thereafter mixed in the desired ratio. This, however, may result in precipitation of one of the components and consequently in workability problems, e.g. in a spraying process. Increased difficulties occur when the mixture comprises a polymer with ionic characteristics, i.e. when one of the polymers to be mixed comprises a monomer having anionic or cationic groups. The presence of such charged groups, however, has a strong effect on the solubility, making it difficult to find a solvent suitable for both the charged polymer and an uncharged polymer.

WO 2006/058779 discloses an enzyme-based sensor with a combined diffusion and enzyme layer comprising at least one polymer material, and particles carry an enzyme, wherein the particles are dispersed in the polymer material. The polymer may comprise hydrophilic as well as hydrophobic polymer chain sequences, for example, the polymer may be a high or low water uptake polyether-polyurethane copolymer. The use of block copolymers having at least one hydrophilic block and at least one hydrophobic block as a diffusion layer is not disclosed.

EP-A-2 163 190 describes an electrode system for the measurement of an analyte concentration *in-vivo* comprising a counterelectrode with an electric conductor, and a working electrode with an electric conductor on which an enzyme layer comprising immobilized enzyme molecules is localized. A diffusion barrier controls the diffusion of the analyte from surrounding body fluids to the enzyme molecules. The diffusion barrier may comprise hydrophilized polyurethanes obtainable by polycondensation of 4,4'-methylene-bis-(cyclohexylisocyanate) and diol mixtures which may be polyethyleneglycol and polypropyleneglycol. The hydrophilic polyurethane layer may be covered with a spacer, e.g. a copolymer of butyl methacrylate and 2-methacryloyloxyethyl-phosphoryl choline. The use of block copolymers having at least one hydrophilic block and at least one hydrophobic block as a diffusion layer is not disclosed. The use of a hydrophilic copolymer of (meth)acrylic monomers comprising more than 50 mol-% hydrophilic monomers is not disclosed either.

It is an object of the present invention to provide a diffusion barrier on an electrode system of an enzymatic *in-vivo* sensor which provides desirable physico-chemical characteristics and which can be manufactured easily.

This object is met by providing a diffusion barrier consisting of a single block copolymer having at least one hydrophilic block and at least one hydrophobic block. The hydrophilic and hydrophobic blocks are covalently linked to each other. Preferably the blocks are (meth)acrylate polymer blocks.

The block-copolymer based diffusion barrier provides excellent physico-chemical characteristics as follows:

(i) permeability of the diffusion barrier for the analyte to be determined,
(ii) permeability characteristics of the diffusion barrier which are suitable for the short-term behaviour (wettability) and the long-term behaviour (sensor drift) of the electrode,
(iii) mechanical flexibility of the diffusion barrier, which allows manufacture of *in-vivo* sensors with extended multiple electrodes;

(iv) efficient incorporation of ionic groups into the diffusion layer, i.e. the density of cationic or anionic charges within the polymer can be efficiently adjusted, this is relevant for repulsion or attraction of

charged analytes, and/or control of cell adhesion, e.g. of monocytes from the surrounding body fluid or tissue.

A subject-matter of the present invention is an electrode system for measuring the concentration of an analyte under *in-vivo* conditions, comprising an electrode with immobilized enzyme molecules and a diffusion barrier that controls diffusion of the analyte from the exterior of the electrode system to the enzyme molecules, characterized in that the diffusion barrier comprises a block copolymer having at least one hydrophilic block and at least one hydrophobic block.

Preferably, the diffusion barrier comprises a single, i.e. only one block copolymer having at least one hydrophilic block and at least one hydrophobic block, i.e. further polymers or copolymers are absent. More preferably, the diffusion barrier consists of a single block copolymer having at least one hydrophilic block and at least one hydrophobic block.

The electrode system of the present invention is suitable for insertion or implantation into a body, e.g. a mammalian body such as a human body. The electrode system is adapted for measuring a desired analyte in body fluid and/or body tissue, e.g. in the extracellular space (interstitium), in blood or lymph vessels or in the transcellular space.

The inserted or implanted electrode system is suitable for short-term application, e.g. 3-14 days, or for long-term application, e.g. 6-12 months. During the insertion or implantation period a desired analyte may be determined by continuous or discontinuous measurements.

The electrode system of the invention is preferably part of an enzymatic, non-fluidic (ENF) sensor, wherein enzymatic conversion of the analyte is determined. Preferably, the sensor comprises a working electrode with immobilized enzyme molecules for the conversion of the analyte which results in the generation of an electrical signal. The enzymes may be present in a layer covering the electrode. Additionally, redox mediators and/or electro-catalysts as well as conductive particles and pore formers may be present. This type of electrode is described e.g. in WO 2007/147475.

The area of the working electrode is the sensitive area of the sensor. This sensitive area is provided with a diffusion barrier that controls diffusion of the analyte from the exterior, e.g. body fluid and/or tissue surrounding the electrode system to the enzyme molecules. The diffusion barrier can, for example, be a cover layer covering the enzyme layer, i.e. an enzyme-free layer. However, it is feasible just as well that diffusion-controlling particles are incorporated into the enzyme layer to serve as a diffusion barrier. For example, pores of the enzyme layer can be filled with the polymer which controls the diffusion of analyte molecules. The thickness of the diffusion barrier is usually from about 2-20 $\mu$m, e.g. from about 2-15 $\mu$m, or from about 5-20 $\mu$m, particularly from about 5-10 $\mu$m or from about 10-15 $\mu$m (in dry state).

The diffusion barrier of the electrode system of the present invention comprises a block copolymer, preferably a single block copolymer having at least one hydrophilic block and at least one hydrophobic block. The block copolymer may comprise an alternating sequence of blocks, i.e. a hydrophilic block is linked to a hydrophobic block. The hydrophilic and hydrophobic blocks are covalently linked to each other within a polymer molecule. The average molecular weight of the polymer (by weight) is usually from 20-70 kD, particularly from 25-60 kD and more particularly from 30-50 kD. The molar ratio of the hydrophilic to hydrophobic portions in the block copolymer is usually in the range from about 75% (hydrophilic) : 25% (hydrophobic) to about 25% (hydrophilic) : 75% (hydrophobic), in the range from about 65% (hydrophilic) : 35% (hydrophobic) to about 35% (hydrophilic) : 65% (hydrophobic) or in the range from about 60% (hydrophilic) : 40% (hydrophobic) to about 40% (hydrophilic) : 60% (hydrophobic). A hydrophilic block of the block copolymer consists of at least 90%, at least 95% and particularly completely of hydrophilic monomeric units. It usually has a length of from 50-400, e.g. from 50-200, or from 150-300 particularly from 100-150, or from 200-250 monomeric molecules. A hydrophobic block of the copolymer consists of at least 90%, more particularly at least 95% and even more particularly completely of hydrophobic monomeric units. It has usually a length of from 50-300, e.g. from 50-200, or from 150-250, particularly from 80-150, or from 170-200 monomeric units.

The hydrophilic blocks and/or the hydrophobic blocks preferably consist of (meth)acrylic-based units. More preferably, both the hydrophilic blocks and the hydrophobic blocks consist of (meth)acrylic-based monomeric units.

The hydrophilic monomeric units of the hydrophilic block are preferably selected from hydrophilic (meth)acryl esters, i.e. esters with a polar, i.e. OH, $OCH_3$ or $OC_2H_5$ group within the alcohol portion of the ester, hydrophilic (meth)acrylamides with an amide ($NH_2$) or an N-alkyl- or N,N-dialkylamide group, wherein the alkyl group comprises 1-3 C-atoms and optionally hydrophilic groups such as OH, $OCH_3$ or $OC_2H_5$, and suitable (meth)acrylic units having a charged, e.g. an anionic or cationic group, such as acrylic acid (acrylate) or methacrylic acid (methacrylate). Further, combinations of monomeric units may be employed.

Specific examples of preferred monomeric units for the hydrophilic block are selected from:

2-hydroxyethyl acrylate,
2-hydroxyethyl methacrylate (HEMA),
2-methoxyethyl acrylate,
2-methoxyethyl methacrylate,

2-ethoxyethyl acrylate,
2-ethoxyethyl methacrylate,
2- or 3-hydroxypropyl acrylate,
2- or 3-hydroxypropyl methacrylate (2- or 3-HPMA),
2- or 3-methoxypropyl acrylate,
2- or 3-methoxypropyl methacrylate,
2- or 3-ethoxypropyl acrylate,
2- or 3-ethoxypropyl methacrylate,
1- or 2-glycerol acrylate,
1- or 2-glycerol methacrylate,
acrylamide,
methacrylamide,
an N-alkyl- or N,N-dialkyl acrylamide, and
an N-alkyl- or N,N-dialkyl methacrylamide, wherein alkyl comprises 1-3 C-atoms such as methyl, ethyl or propyl,
acrylic acid (acrylate),
methacrylic acid (methacrylate)
and combinations thereof.

Preferred hydrophilic monomers are 2-hydroxyethyl methacrylate (HEMA) and/or 2- or 3-hydroxypropyl methacrylate (2- or 3-HPMA). More preferably, the hydrophilic block consists of at least two different hydrophilic monomeric units. For example, it may be a random copolymer of at least two different hydrophilic monomeric units such as HEMA and 2-HPMA.

In order to introduce ionic groups into the monomer, charged monomeric units such as acrylic acid (acrylate) and/or methacrylic acid (methacrylate) may be incorporated into the hydrophilic block. Thus, in a particular embodiment of the present invention, the hydrophilic block can be made from at least one non-ionic hydrophilic monomeric unit (e.g. as described above) and from at least one ionic hydrophilic monomeric unit, wherein the ionic monomeric unit is present in a molar amount of preferably 1-20 mole-%. In case the hydrophilic block comprises an ionic monomeric unit such as acrylic acid or methacrylic acid, copolymerization with a hydrophilic monomer selected from the group of (meth)acrylamides, particularly N,N-dialkyl acryl- or methacrylamides is preferred.

The hydrophobic monomeric units of the hydrophobic block are preferably selected from hydrophobic acrylic and/or methacrylic units, styrene-based monomeric units or combinations thereof. Preferably the hydrophobic monomeric units are selected from hydrophobic (meth)acryl esters, e.g. esters having an alcohol portion with 1-3 C-atoms without hydrophilic group. Specific examples of monomeric units for the hydrophobic block are selected from:

methyl acrylate,
methyl methacrylate (MMA),
ethyl acrylate
ethyl methacrylate (EMA),
n- or i-propyl acrylate,
n- or i-propyl methacrylate,
n-butyl acrylate,
n-butyl methacrylate (BUMA),
neopentyl acrylate,
neopentyl methacrylate,
and combinations thereof.

The hydrophobic block preferably comprises at least two different hydrophobic monomeric units, which are e.g. present as a random copolymer. In a preferred embodiment, the hydrophobic block comprises methyl methacrylate (MMA) and n-butyl methacrylate (BUMA). In an especially preferred embodiment, the hydrophobic block is a random copolymer of MMA and BUMA. The molar ratio between MMA and BUMA is preferably about 60% (MMA) : 40% (BUMA) to about 40% (MMA) : 60% (BUMA), e.g. about 50% (MMA) : 50% (BUMA). The glass transition temperature of the hydrophobic block is preferably 100°C or less, 90°C or less or 80°C or less, e.g. about 40-80°C. In an alternative embodiment, the hydrophobic block may consist of styrenic units, e.g. of polystyrene having a glass transition temperature of about 95°C.

The block copolymers used in the present invention may be manufactured according to known methods (Böker et al., Macromolecules 34 (2001), 7477-7488).

The block copolymers may be applied to the electrode system by usual techniques, e.g. by providing a solution of the block copolymer in a suitable solvent or solvent mixture, e.g. an organic solvent, such as ether, which is applied to the prefabricated electrode system and dried thereon.

When the block copolymer is contacted with water, it shows a water uptake of preferably about 15%-30% by weight (based on the polymer dry weight) at a temperature of 37°C and a pH of 7.4 (aqueous phosphate buffer 10 mM $KH_2PO_4$, 10 mM $NaH_2PO_4$ and 147 mM NaCl).

In addition to the block copolymer the diffusion barrier may also comprise further components, particularly non-polymeric components, which may be dispersed and/or dissolved in the polymer. These further compounds include plasticizers, particularly biocompatible plasticizers, such as tri-(2-ethylhexyl) trimellitate and/or glycerol.

The diffusion barrier of the invention has a high effective diffusion coefficient $D_{eff}$ for glucose which is preferably $\geq 10^{-10}$ cm$^2$/s, more preferably $\geq 5\cdot10^{-10}$ cm$^2$/s, and even more preferably $\geq 10^{-9}$ cm$^2$/s, and e.g. up to $10^{-7}$ or $10^{-8}$ cm$^2$/s at a temperature of 37°C and a pH of 7.4. The effective diffusion coefficient is preferably determined as described in Example 4 according to the equation:

$$D_{eff}=SE_m/F\cdot L_m\cdot 5,182\cdot10^{-8}$$

wherein $SE_m$ is the sensitivity of the working electrode, F is the area of the working electrode, and $L_m$ is the layer thickness of the diffusion barrier. $SE_m$ and $L_m$ may be determined as described in the Examples.

The electrode system of the present invention is suitable for measuring the concentration of an analyte under in-vivo conditions, i.e. when inserted or implanted into a body. The analyte may be any molecule or ion present in tissue or body fluid, for example oxygen, carbon dioxide, salts (cations and/or anions), fats or fat components, carbohydrates or carbohydrate components, proteins or protein components, or other type of biomolecules. Especially preferred is the determination of analytes which can be efficiently transferred between body fluid, e.g. blood and tissue such as oxygen, carbon dioxide, sodium cations, chloride anions, glucose, urea, glycerol, lactate and pyruvate.

The electrode system comprises an enzyme immobilized on an electrode. The enzyme is suitable for the determination of a desired analyte. Preferably, the enzyme is capable of catalytically converting the analyte and thereby generating an electric signal detectable by the electric conductor of the working electrode. The enzyme for measuring the analyte is preferably an oxidase, for example glucose oxidase or lactate oxidase or a dehydrogenase, for example a glucose dehydrogenase or a lactate dehydrogenase. In addition to the enzyme, the enzyme layer may also comprise an electrocatalyst or a redox mediator which favours the transfer of electrons to conductive components of the working electrode, e. g. graphite particles. Suitable electro-catalysts are metal oxides such as manganese dioxide or organo-metallic compounds such as cobalt phthalo-cyanine. In a preferred embodiment the redox mediator is capable of degrading hydrogen peroxide thereby counteracting depletion of oxygen in the surroundings of the working electrode. In a different embodiment, a redox mediator may be covalently bound to the enzyme and thereby effect direct electron transfer to the working electrode. Suitable redox mediators for direct electron transfer are prosthetic groups, such as pyrrolo quinoline quinone (PQQ), flavine adenine dinucleotide (FAD) or other known prosthetic groups. Enzymes immobilized on electrodes are e.g. described in WO 2007/147475.

A preferred embodiment of the electrode system comprises a counterelectrode with an electrical conductor and a working electrode with an electrical conductor on which an enzyme layer and the diffusion barrier are arranged. The enzyme layer is preferably designed in the form of multiple fields that are arranged on the conductor of the working electrode at a distance, e.g. at least 0.3 mm or at least 0.5 mm from each other. The individual fields of the working electrode may form a series of individual working electrodes. Between these fields, the conductor of the working electrode may be covered by an insulation layer. By arranging the fields of the enzyme layer on the top of openings of an electrically insulating layer, the signal-to-noise ratio can be improved. Such an arrangement is disclosed in WO 2010/028708.

The electrode system of the invention may additionally comprise a reference electrode capable of supplying a reference potential for the working electrode, e.g. an Ag/Ag-Cl reference electrode. Moreover, an electrode system according to the invention can have additional counter- and/or working electrodes.

The electrode system may be part of a sensor, e.g. by being connected to a potentiostat and an amplifier for amplification of measuring signals of the electrode system. The sensor is preferably an enzymatic non-fluidic (ENF) sensor, more preferably an electrochemical ENF sensor The electrodes of the electrode system may be arranged on a substrate that carries the potentiostat or be attached to a circuit board that carries the potentiostat.

A further subject-matter of the invention is related to the use of a block copolymer having at least one hydrophilic block and at least one hydrophobic block as a diffusion barrier for an enzymatic electrode. The block copolymer is preferably as described above, e.g. a single block-copolymer. The diffusion barrier and the enzymatic electrode are preferably also as described above.

Further details and advantages of the invention are explained based on an exemplary embodiment making reference to the appended drawings.

**Fig. 1** shows an exemplary embodiment of an electrode system according to the invention.

**Fig. 2** shows a detail view of Fig. 1.

**Fig. 3** shows another detail view of Fig. 1.

**Fig. 4** shows a section along the section line CC of Fig. 2.

**Fig. 5** shows the sensitivity (with standard deviation) of four glucose sensors (at 10 mM glucose) provided with different block polymers (C, F, D, B) as barrier layers.

**Fig. 6** shows the sensor drift of four glucose sensors provided with different block copolymers (A, C, D, F) as barrier layers.

**Fig. 7** shows the conductivity of block copolymer A dependent on time (2 experiments).

**Fig. 8** shows the conductivity of block copolymer F dependent on time (3 experiments).

**Fig. 9** shows the conductivity of block copolymer H dependent on time for a layer thickness of 2.77 $\mu$m or 4.43 $\mu$m, respectively.

**Fig. 10** shows the fibrinogen adhesion to different spacer membrane polymers *in-vitro* (Adapt® and Eudragit E100) with respect to an uncoated plate (Blank).

**Fig. 11** shows the expression of surface protein CD54 by THP-1 cells after incubation with sensors coated with a spacer membrane (Adapt® and Lipidure CM5206) or uncoated (control = untreated cells).

**Fig. 12a** and **12b** show the secretion of cytokine IL-8 and MCP-1, respectively, by THP-1 cells after incubation with sensors coated with a spacer membrane (Adapt® and Lipidure CM5206) or uncoated (control = untreated cells).

**Fig. 13** shows the secretion of cytokine IL-8 by THP-1 cells after incubation with tissue culture plates coated with a spacer membrane (Adapt®, Lipidure CM5206 and Eudragit E100) or uncoated (Polyst.), and an additional layer of fibrinogen.

**Fig. 14** shows the haemolysis after incubation with sensors coated with a spacer membrane (Adapt® and Lipidure CM5206) or uncoated in comparison to the spacer polymer Adapt® without sensor (negative control = incubation medium only; positive control = 100% osmotic lysis).

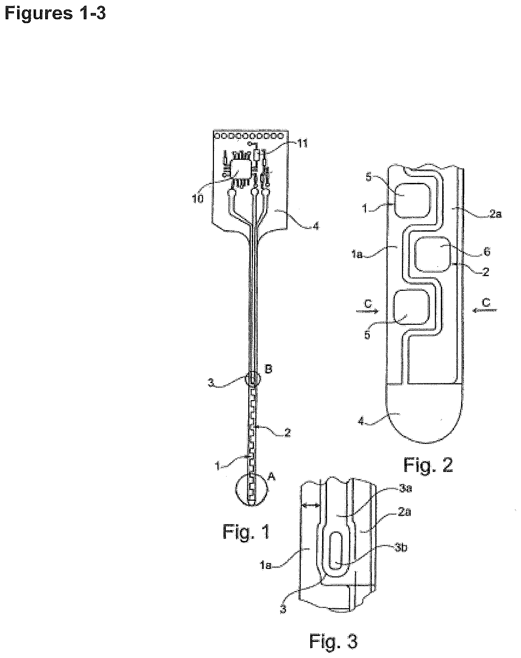

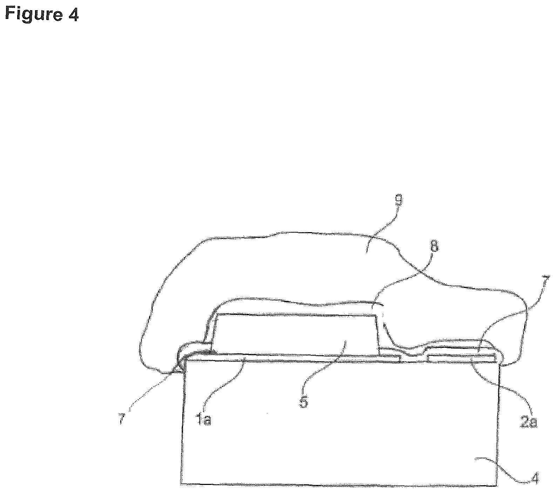

Figure 1 shows an exemplary embodiment of an electrode system for insertion into body tissue of a human or animal, for example into cutis or subcutaneous fatty tissue. A magnification of detail view A is shown in Figure 2, a magnification of detail view B is shown in Figure 3. Figure 4 shows a corresponding sectional view along the section line, CC, of Figure 2.

The electrode system shown has a working electrode 1, a counterelectrode 2, and a reference electrode 3. Electrical conductors of the electrodes 1 a, 2a, 3a are arranged in the form of metallic conductor paths, preferably made of palladium or gold, on a substrate 4. In the exemplary embodiment shown, the substrate 4 is a flexible plastic plate, for example made of polyester. The substrate 4 is less than 0.5 mm thick, for example 100 to 300 micrometers, and is therefore easy to bend such that it can adapt to movements of surrounding body tissue after its insertion. The substrate 4 has a narrow shaft for insertion into body tissue of a patient and a wide head for connection to an electronic system that is arranged outside the body. The shaft of the substrate 4 preferably is at least 1 cm in length, in particular 2 cm to 5 cm.

In the exemplary embodiment shown, one part of the measuring facility, namely the head of the substrate, projects from the body of a patient during use. Alternatively, it is feasible just as well, though, to implant the entire measuring facility and transmit measuring data in a wireless fashion to a receiver that is arranged outside the body.

The working electrode 1 carries an enzyme layer 5 that contains immobilized enzyme molecules for catalytic conversion of the analyte. The enzyme layer 5 can be applied, for example, in the form of a curing paste of carbon particles, a polymeric binding agent, a redox mediator or an electro-catalyst, and enzyme molecules. Details of the production of an enzyme layer 5 of this type are disclosed, for example, in WO 2007/147475, reference to which is made in this context.

In the exemplary embodiment shown, the enzyme layer 5 is not applied continuously on the conductor 1a of the working electrode 1, but rather in the form of individual fields that are arranged at a distance from each other. The individual fields of the enzyme layer 5 in the exemplary embodiment shown are arranged in a series.

The conductor 1 a of the working electrode 1 has narrow sites between the enzyme layer fields that are seen particularly well in Figure 2. The conductor 2a of the counterelectrode 2 has a contour that follows the course of the conductor 1a of the working electrode 1. This means results in an intercalating or interdigitated arrangement of working electrode 1 and counterelectrode 2 with advantageously short current paths and low current density.

In order to increase its effective surface, the counterelectrode 2 can be provided with a porous electrically conductive layer 6 that is situated in the form of individual fields on the conductor 2a of the counterelectrode 2. Like the enzyme layer 5 of the working electrode 1, this layer 6 can be applied in the form of a curing paste of carbon particles and a polymeric binding agent. The fields of the layer 6 preferably have the same dimensions as the fields of the enzyme layer 5, although this is not obligatory. However, measures for increasing the surface of the counterelectrode can just as well be foregone and the counterelectrode 2 can just as well be designed to be a linear conductor path with no coatings of any kind, or with a coating made from the described block copolymer and optionally a spacer.

The reference electrode 3 is arranged between the conductor 1 a of the working electrode 1 and the conductor 2a of the counterelectrode 2. The reference electrode shown in Figure 3 consists of a conductor 3a on which a field 3b of conductive silver/silver chloride paste is arranged.

Figure 4 shows a schematic sectional view along the section line, CC, of Figure 2. The section line, CC, extends through one of the enzyme layer fields 5 of the working electrode 1 and between the fields of the conductive layer 6 of the counterelectrode 2. Between the fields of enzyme layer 5, the conductor 1a of the working electrode 1 can be covered with an electrically insulating layer 7, like the conductor 2a of the counterelectrode 2 between the fields of the conductive layers 6, in order to prevent interfering reactions which may otherwise be catalysed by the metal of the conductor paths 1 a, 2a. The fields of the enzyme layer 5 are therefore situated in openings of the insulation layer 7. Likewise, the fields of the conductive layer 6 of the counterelectrode 2 may also be placed on top of openings of the insulation layer 7.

The enzyme layer 5 is covered by a cover layer 8 which presents a diffusion resistance to the analyte to be measured and therefore acts as a diffusion barrier. The diffusion barrier 8 consists of a single copolymer with alternating hydrophilic and hydrophobic blocks as described above.

A favourable thickness of the cover layer 8 is, for example, 3 to 30 $\mu$m, particularly from about 5-10 $\mu$m or from about 10-15 $\mu$m. Because of its diffusion resistance, the cover layer 8 causes fewer analyte molecules to reach the enzyme layer 5 per unit of time. Accordingly, the cover layer 8 reduces the rate at which analyte molecules are converted, and therefore counteracts a depletion of the analyte concentration in surroundings of the working electrode.

The cover layer 8 extends continuously essentially over the entire area of the conductor 1 a of the working electrode 1. On the cover layer 8, a biocompatible membrane may be arranged as spacer 9 that establishes a minimal distance between the enzyme layer 5 and cells of surrounding body tissue. This means advantageously generates a reservoir for analyte molecules from which analyte molecules can get to the corresponding enzyme layer field 5 in case of a transient disturbance of the fluid exchange in the surroundings of an enzyme layer field 5. If the exchange of body fluid in the surroundings of the electrode system is transiently limited or even prevented, the analyte molecules stored in the spacer 9 keep diffusing to the enzyme layer 5 of the working electrode 1 where they are converted. The spacer 9 therefore causes a notable depletion of the analyte concentration and corresponding falsification of the measuring results to occur only after a significantly longer period of time. In the exemplary embodiment shown, the membrane forming the spacer 9 also covers the counterelectrode 2 and the reference electrode 3.

The spacer membrane 9 can, for example, be a dialysis membrane. In this context, a dialysis membrane is understood to be a membrane that is impermeable for molecules larger than a maximal size. The dialysis membrane can be prefabricated in a separate manufacturing process and may then be applied during the fabrication of the electrode system. The maximal size of the molecules for which the dialysis membrane is permeable is selected such that analyte molecules can pass, while larger molecules are retained.

Alternatively, instead of a dialysis membrane, a coating made of a polymer that is highly permeable for the analyte and water, for example on the basis of polyurethane or of acrylate, can be applied over the electrode system as spacer membrane 9.

Preferably, the spacer is made from a copolymer of (meth)acrylates. Preferably, the spacer membrane is a copolymer from at least 2 or 3 (meth)acrylates. More preferably, the spacer membrane comprises more than 50 mol-%, at least 60 mol-% or at least 70 mol-% hydrophilic monomer units, e.g. HEMA and/or 2-HPMA, and up to 40 mol-% or up to 30 mol-% hydrophobic units, e.g. BUMA and/or MMA. The spacer may be a random or block copolymer. An especially preferred spacer membrane comprises MMA or BUMA as hydrophobic moieties and 2-HEMA and/or 2-HPMA as hydrophilic moieties. Preferably, the amount of the hydrophilic monomers HEMA and/or HPMA is between 80 mol-% to 85 mol-% and the amount of hydrophobic component MMA and/or BUMA is between 15 mol-% and 20 mol-%.

The very preferred spacer membrane of the invention is made of the copolymer Adapt® (BioInteractions Ltd, Reading, England). Adapt® comprises BUMA as hydrophobic moiety and 2-HEMA and 2-HPMA as hydrophilic moieties, wherein the amount of the 2-HEMA hydrophilic monomers is about 80 mol-%.

The spacer membrane is highly permeable for the analyte, i.e. it does significantly lower the sensitivity per area of the working electrode, for example 20% or less, or 5% or less with a layer thickness of less than about 20 $\mu$m, preferably less than about 5 $\mu$m. An especially preferred thickness of the spacer membrane is from about 1 to about 3 $\mu$m.

The enzyme layer 5 of the electrode system can contain metal oxide particles, preferably manganese dioxide particles, as catalytic redox mediator. Manganese dioxide catalytically converts hydrogen peroxide that is formed, for example, by enzymatic oxidation of glucose and other bioanalytes. During the degradation of hydrogen peroxide, the manganese di-oxide particles transfer electrons to conductive components of the working electrode 1, for example to graphite particles in the enzyme layer 5. The catalytic degradation of hydrogen peroxide counteracts any decrease of the oxygen concentration in the enzyme layer 5. Advantageously this allows the conversion of the analyte to be detected in the enzyme layer 5 to not be limited by the local oxygen concentration. The use of the catalytic redox mediator therefore counteracts a falsification of the measuring result by the oxygen concentration being low. Another advantage of a catalytic redox mediator is that it prevents the generation of cell-damaging concentrations of hydrogen peroxide.

The preferred spacer membrane polymer described herein may be used as an outer coating for a diffusion barrier of the present invention, but also as an outer coating of an electrode system in general, particularly of an electrode system for measuring the concentration of an analyte under *in-vivo* conditions, comprising an electrode with immobilized enzyme molecules and a diffusion barrier that controls diffusion of the analyte from the exterior of the electrode system to the enzyme molecules. Thus, the spacer membrane can be arranged on the diffusion barrier, however, the spacer membrane can also be arranged directly on the enzyme layer. In this last context, the spacer membrane can also act as a diffusion barrier itself and slow down the diffusion of analyte molecules to the enzyme layer.

When the electrode system of the invention is inserted or implanted into a body, the spacer membrane is the interface between the implanted sensor and the surrounding body fluid or tissue. Consequently, when exposed to the body fluid or tissue, the spacer membrane of the invention must be mechanically robust so that it is neither deformed nor shoved off the sensor. To this end, the spacer membrane copolymer water uptake and the concomitant swelling of the copolymer must be limited, albeit the inherent hydrophilicity of the copolymer.

Preferably, the relative water uptake of the spacer membrane copolymer should not exceed 50 wt-%, preferably 40 wt-%, more preferably 30 wt-% based on the total rate of the copolymer. Within the context of the present invention, the measurement of the relative water uptake is performed by subjecting the dry copolymer to an excess of phosphate-buffer (pH 7.4) for 48 h at a temperature of 37 °C. The relative water uptake (WU%) is preferably determined according to the equation:

$$WU\% = (m_2-m_1)/m_1 \times 100,$$

wherein $m_1$ and $m_2$ represent, respectively, the mass of the dry copolymer and the copolymer after hydration according to the above measurement conditions.

The inventors of the present invention determined that the preferred spacer membrane made of the copolymer Adapt® takes up 33 $\pm$ 1.8 wt-% of phosphate-buffer (pH 7.4) relative to its own weight over 48 h at 37° C. Under the same conditions, a membrane of polymer Lipidure CM5206 (NOF Corporation, Japan) takes up 157 $\pm$ 9.7 wt-% of phosphate-buffer relative to its own weight. The lower water uptake of the polymer advantageously increases the mechanical stability of the spacer membrane of the present invention. By contrast, Lipidure CM5206 shows a higher water uptake and swells to a hydrogel which is more fragile, easily deformable or shoved off, particularly when applied on an electrode system of an enzymatic *in-vivo* sensor.

Furthermore, during insertion and implementation of the electrode system, the spacer is in direct contact with the tissue and/or the body fluid, like interstitial fluid or blood, containing biomolecules like proteins and cells. Advantageously, the spacer membrane must protect the inserted and implanted sensor in the tissue and/or body fluid environment and, thus, minimize the tissue reaction of the body to the implant. In fact, reactions of the body against implanted material are known as "*foreign body response*" (FBR). By FBR, the body tries to destroy the implant or, if it is not possible, to create a capsule to separate it from the surrounding tissue (foreign body granuloma). The first step of the FBR reaction is binding of proteins (e.g. fibrinogen, albumin, immunoglobulin, complement) to the surface of the former material, i.e. the implant. This protein coat presents binding sites to receptors on immune cells. For example, fibrinogen contains a structural motif that binds to the monocyte receptor MAC-1. When fibrinogen binds to the surface of the implant, it changes its conformation and exposes the binding site for MAC-1. Consequently, immune cells, like monocytes, are recruited to the implant and activated, secreting enzymes and radicals to attack the implant. Additionally, immune cells secrete soluble factors, i.e. cytokines, to recruit and activate other immune cells and thereby amplifying the immune response. If the implant cannot be removed, a fibrous capsule is formed by connective tissue cells and proteins. This capsule, however, is a diffusion barrier for analytes to reach the sensor. Collectively, the events of the foreign body response as described above are likely to interfere with the electrode system function *in-vivo* and with its life time.

Thus, an improved spacer membrane on an electrode-system of an enzymatic *in-vivo* sensor further provides the reduction of the tissue response to the implant and inhibits the formation of a capsule separating the sensor from the surrounding tissue and body fluids.

Thus, it is a further object of the present invention to provide an electrode system for measuring the concentration of an analyte under *in-vivo* conditions, comprising an electrode with immobilized enzyme molecules and preferably a diffusion barrier that controls diffusion of the analyte form the exterior of the electrode system to the enzyme molecules, characterised in that a spacer membrane forms at least a portion of the outer layer of the electrode system, wherein the spacer membrane comprises a hydrophilic copolymer of acrylic and/or methacrylic monomers, wherein the polymer comprises more than 50 mol-% hydrophilic monomers.

As described above, the spacer membrane of the invention does have limited protein-binding capacity to protect the electrode system of the sensor from protein adsorption that might trigger response of immune cells and might limit or interfere with its performance *in-vivo.* Example 5 and 6 show that the preferred spacer membrane of the invention provides little binding to fibrinogen and prevents conformational change of fibrinogen that would lead to the exposure of the MAC-1 binding motif for monocytes. Advantageously, the spacer membrane copolymer material does not activate immune cells itself. In Example 6, it could be demonstrated that the spacer membrane copolymer of the invention is able to attenuate the activation of immune cells by the implanted sensor. Moreover, advantageously, the spacer membrane is a biocompatible material, in particular, is compatible with body fluids, e.g. with blood. Example 7 shows that the spacer membrane copolymer of the present invention is able to prevent haemolysis and the complement activation by the implanted sensor. Thus, the spacer membrane of the invention advantageously not only shows a high mechanical stability, but also has optimal biocompatible properties, which is surprising due to the low water uptake when wetted.

The features of this embodiment particularly with regard to the structure of the electrode system, the analyte and the enzyme molecules are as described herein. The diffusion barrier is preferably as described herein, it may however also have a different composition or may be absent. According to one preferred embodiment, the diffusion barrier preferably comprises a block copolymer having at least one hydrophilic block and at least one hydrophobic block as described herein.

According to a further preferred embodiment, the diffusion barrier comprises hydrophilic polyurethanes. The hydrophilic polyurethanes used as diffusion membrane can be produced by polyaddition of an (poly)diisocyanat, preferably 4-4-methylene-bis(cyclohexylisocyanate) with a polyalcohol, preferably a diol mixture.

The components of the diol mixture are preferably polyalkylene glycols, such as polyethylene glycol (PEG) and polypropylene glycol (PPG) and aliphatic diols, such as ethylene glycol. Preferably, the hydrophilic polyurethane comprises 45-55 mol%, preferably 50 mol-% isocyanate and 25-35 mol-%, preferably 30 mol-% ethylene glycol. The degree of hydrophilization is then adjusted by the ratio of PEG to PPG. Preferably, the polyurethane comprises 2-3 mol-%, more preferably 2.5 mol-% PEG and 17-18 mol-%, preferably 17,5 mol-% PPG. In order to increase the hydrophility of the polyurethane, the proportion of PEG may be increased, for instance, to 4.5 - 5.5 mol-%, preferably 5 mol-% PEG, in order to obtain an extremely hydrophilic polyurethane. The different hydrophilic variants of the polyurethanes may also be mixed in order to optimize the properties of the diffusion barrier.

The preferred acrylic and methacrylic monomers of the spacer membrane copolymer are as described herein.

The hydrophilic monomeric units are preferably selected from hydrophilic (meth)acryl esters, i.e. esters with a polar, i.e. OH, $OCH_3$ or $OC_2H_5$ group within the alcohol portion of the ester, hydrophilic (meth)acrylamides with an amide ($NH_2$) or an N-alkyl- or N,N-dialkylamide group, wherein the alkyl group comprises 1-3 C-atoms and optionally hydrophilic groups such as OH, $OCH_3$ or $OC_2H_5$, and suitable (meth)acrylic units having a charged, e.g. an anionic or cationic group, such as acrylic acid (acrylate) or methacrylic acid (methacrylate). Further, combinations of monomeric units may be employed.

Specific examples of preferred monomeric units for the hydrophilic block are selected from:

2-hydroxyethyl acrylate,
2-hydroxyethyl methacrylate (HEMA),
2-methoxyethyl acrylate,
2-methoxyethyl methacrylate,
2-ethoxyethyl acrylate,
2-ethoxyethyl methacrylate,
2- or 3-hydroxypropyl acrylate,
2- or 3-hydroxypropyl methacrylate (2- or 3-HPMA),
2- or 3-methoxypropyl acrylate,
2- or 3-methoxypropyl methacrylate,
2- or 3-ethoxypropyl acrylate,
2- or 3-ethoxypropyl methacrylate,
1- or 2-glycerol acrylate,

1- or 2-glycerol methacrylate,
acrylamide,
methacrylamide,
an N-alkyl- or N,N-dialkyl acrylamide, and
an N-alkyl- or N,N-dialkyl methacrylamide, wherein alkyl comprises 1-3 C-atoms such as methyl, ethyl or propyl,
acrylic acid (acrylate),
methacrylic acid (methacrylate)
and combinations thereof.

Preferred hydrophilic monomers are 2-hydroxyethyl methacrylate (HEMA) and/or 2- or 3-hydroxypropyl methacrylate (2- or 3-HPMA).

The hydrophobic monomeric units are preferably selected from hydrophobic acrylic and/or methacrylic units or combinations thereof. Preferably the hydrophobic monomeric units are selected from hydrophobic (meth)acryl esters, e.g. esters having an alcohol portion with 1-3 C-atoms without hydrophilic group.

Specific examples of monomeric units for the hydrophobic block are selected from:

methyl acrylate,
methyl methacrylate (MMA),
ethyl acrylate
ethyl methacrylate (EMA),
n- or i-propyl acrylate,
n- or i-propyl methacrylate,
n-butyl acrylate,
n-butyl methacrylate (BUMA),
neopentyl acrylate,
neopentyl methacrylate,
and combinations thereof.

In a preferred embodiment, the hydrophobic block comprises methyl methacrylate (MMA) and n-butyl methacrylate (BUMA).

The outer spacer membrane preferably covers at least the working electrode portion comprising the enzyme molecules and optionally also other portions, e.g. the counter electrode. If one is present, the spacer membrane also covers the reference electrode. The spacer membrane preferably covers the entire implanted surface of the electrode system. The spacer membrane preferably covers the working electrode, optionally the counter-electrode and the reference electrode if present in the form of a continuous layer.

The electrode system comprising the improved spacer membrane of the invention may be part of a sensor, e.g. by being connected to a potentiostat and an amplifier for amplification of measuring signals of the electrode system. The sensor is preferably an enzymatic non-fluidic (ENF) sensor, more preferably an electrochemical ENF sensor. The electrodes of the electrode system may be arranged on a substrate that carries the potentiostat or be attached to a circuit board that carries the potentiostat. Preferably, the sensor is for the measurement of glucose.

A further subject-matter of the invention is related to the use of a hydrophilic copolymer of acrylic and/or methacrylic monomers, wherein the hydrophilic copolymer comprises more than 50 mol-% hydrophilic monomers as a spacer membrane for an enzymatic electrode. The hydrophilic copolymer is preferably as described above. Preferably the spacer membrane is used for minimising the foreign body reaction (FRB) against the enzymatic electrode when it is inserted or implanted into the body.

**Example 1** Permeability of an enzymatic non-fluidic (ENF) glucose sensor with distributed electrodes for transcutaneous implantation having a diffusion layer consisting of one single block copolymer.

The sensor was built on a prefabricated palladium strip conductor structure on a polyester substrate having a thickness of 250 $\mu$m. Working electrode (WE) and counterelectrode (CE) were arranged distributedly (as shown in Figs 1-2).

The fields of the CE were overprinted with carbon paste, the rest of the strip conductor was insulated. The fields of the WE were overprinted with a mixture of cross-linked glucose oxidase (enzyme), conductive polymer paste and electric catalyst, here manganese(IV)-oxide (Technipur). The remaining paths of the strip conductor were again insulated. The reference electrode (RE) consists of Ag/AgCl paste. The electrodes cover about 1 cm of the sensor shaft.

The WE-fields were coated with a block copolymer diffusion layer consisting of a HEMA block and a BUMA block. The thickness of the layer is 7 $\mu$m.

Four sensor batches were produced, each provided with a specific block copolymer as diffusion layer (see list herein below). All block copolymers were obtained from Polymer Source, Montreal and are listed in the following

Table 1.

| Name | molecular ratio/% | monomeric units | molecular weight |
| --- | --- | --- | --- |
| Copolymer | BUMA/HEMA | HEMA | Copolymer [kD] |
| C | 73/27 | 92 | 47 |
| F | 60/40 | 108 | 37 |
| D | 48/52 | 162 | 44 |
| B | 62/38 | 169 | 61 |

The respective block copolymer was dissolved in organic solvent (25% concentration) and the sensors were coated therewith. After drying by means of belt driers (2 min, 30 to 50°C), the coated sensors were tested *in-vitro* in glucose solutions of different concentrations. Of each sensor batch 10 sensors were measured as random sample. As a measure for the *in-vitro* sensitivity, the signal was calculated by the difference of the measured currents at 10 mM and 0 mM glucose concentration, which then was divided by 10 mM (cf. Example 4).

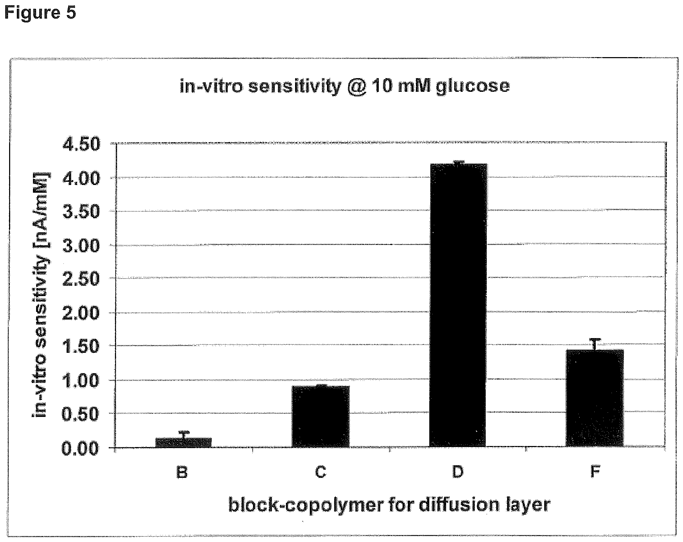

All sensors were operated at a polarisation voltage of 350 mV versus Ag/AgCl, the measured temperature was kept constant at 37°C. The sensors used for this measurement series did not comprise the spacer described in WO 2010/028708, which, however, did not make any difference in view of the tested signal level. Fig. 5 shows the sensor sensitivity with standard deviations for the four different diffusion layers.

Concerning block copolymers C, D and F, there is a clear connection between *in-vitro* sensitivity and molar ratio of hydrophobic block compared to hydrophilic block. At about identical total chain length of the copolymer, the sensitivity increases as the amount of hydrophilic block (HEMA) increases.

The sensors having a diffusion layer of block copolymer B are an exception. Even though polymer B has a relative ratio of hydrophobic to hydrophilic amount similar to polymer F, the sensitivity and thus the permeability for glucose is reduced. Empirically it can be stated that in case of polymer B the total chain length - corresponding to the molecular weight (total) of the copolymer molecule - is so large that the permeability of the layer is reduced. This may also be seen in the gravimetrically determined water uptake of block copolymer B as compared to the remaining polymers. Polymer B has a water uptake of 10.6%±1.5% (weight percent referred to the polymer dry weight). Polymer C lies at 15.6%±0.0%, polymer F at 16.5±3.1% and polymer D at 27%±1.7%.

**Example 2** Mechanic flexibility of the diffusion layer of an ENF glucose sensor

The sensor was manufactured as described in WO2010/028708, however having a diffusion layer according to the present invention. It was assumed that the glass transition temperature (Tg) is a substitute parameter for the mechanic flexibility. Further, it was assumed that the glass transition temperature, which may be allocated to the hydrophobic block, determines the mechanic flexibility in *in-vivo* applications. It should be noted that several Tgs may be identified for one block copolymer, corresponding to the number of blocks.

The sensors were coated with the same electrode pastes as in Example 1. Then, some of the sensors were coated with a copolymer selected from MMA-HEMA (produced by Polymer Source, Montreal). This polymer (called E) has a total molecular weight of 41 kD, the molar ratio of MMA (hydrophobic amount) to HEMA is 60%:40%. The glass transition temperature of the hydrophobic block is 111°C, determined by DSC and a heating rate of 10°C/min.

Besides, other sensors were provided with a diffusion layer of a block copolymer of the invention (called A). The hydrophobic block of said copolymer A contains MMA and BUMA at equal molar amounts in a randomised sequence. Again, the molar ratio of the hydrophobic part to the hydrophilic part is 60%:40%. The molecular weight is 36 kD. The Tg of the hydrophobic block decreases, due to the randomized sequence of MMA and BUMA (Tg about 45°C), to 73°C.

Both diffusion layers were generated from the respective solution (25%) of the copolymers in ether and dried as in **Example 1.** The thickness of the diffusion layers was 7 μm. A spacer layer was applied subsequently via dip coating and dried 24h at room temperature. The spacer layer was made of Lipidure CM 5206, produced by NOF Japan.

After explantation from the tissue, sensors having a copolymer E diffusion layer show sporadic cracks in the diffusion layer. This is taken as an effect of the mechanic load. In contrast thereto, sensors having a copolymer A diffusion layer, do not show any cracks under identical load. This is obviously due to the reduction of Tg, which increases the mechanic stability of the copolymer. A physical mixture of two copolymers, as disclosed in WO2010/028708, is no longer required.

**Example 3** Optimized permeation behaviour of an ENF glucose sensor with distributed electrode and diffusion layer according to the invention.

A sensor was manufactured as described in **Example 1,** but with an additional spacer layer on the total of the sensor shaft. Sensors with respective diffusion layer were produced for copolymers A, C, D and F of **Examples 1 and 2.** For this purpose, a 24% etheric solution of the copolymer was generated. Each solution was applied onto a set of sensors (N=10) and then dried in a band drier. Thereby, diffusion layers having a thickness of $7\,\mu m$, were obtained.

Afterwards, the sensors were provided with a spacer layer as described in **Example 2.**

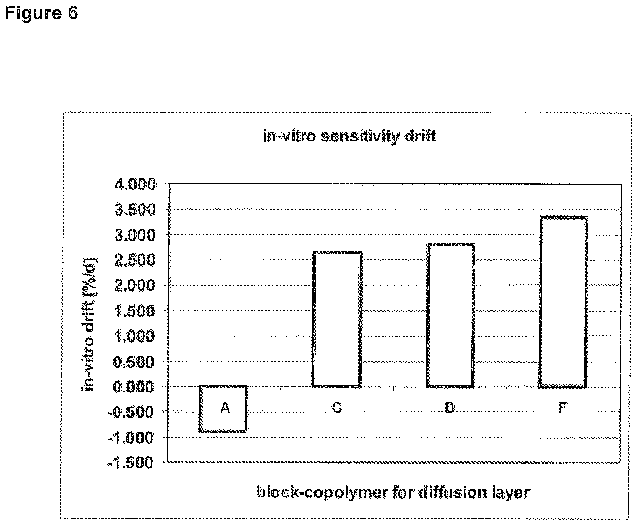

The sensor was connected with a measuring system on the sensor head, which transfers the measured data to a data store. The *in-vitro* measurements were carried out as in **Example 1,** however over a measuring period of 7 days. From the measured data, the sensitivity drift was calculated over the respective measuring period for each sensor. Figure 6 shows for each sensor variant, i.e. sensors of a variant of the diffusion layer, the mean value of the *in-vitro* drift value for the group. The initial phase of the measurement - the first 6h, the so-called startup phase - was excluded from the calculation.

For all copolymers C, D and F having a hydrophobic block of BUMA, there is a positive drift, i.e. the sensitivity increases according to time. Contrary thereto, copolymer A with the hydrophobic block of a random copolymer of MMA and BUMA, leads to a very low, slightly negative, drift.

These differences may be explained by the long-time permeability response of the respective diffusion layers, which was measured in additional experiments. Palladium sensors without WE-paste, but with a defined active surface, i.e. also without an enzyme layer - excluding the influence of its swelling behaviour on the results - were coated with the above polymer solutions, and after drying, the thickness of the layer was measured. Subsequently, conductivity was measured in sodium- and chloride-containing buffer solution.

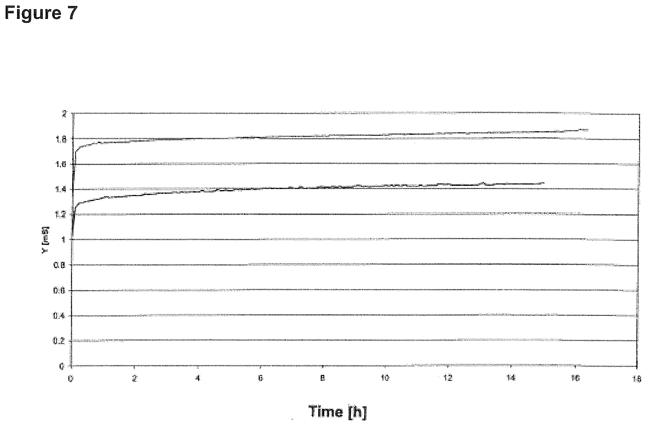

Fig. 7 shows that the conductivity of copolymer A remained nearly constant after a short startup phase.

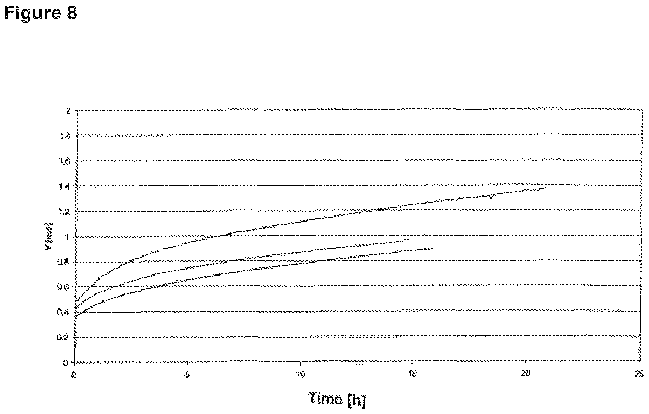

This is not the case for copolymer F, even under identical measurement conditions, as may be seen in Fig. 8. In this case, a long-term and strong permeability response of the diffusion layer of copolymer F was observed, which was practically independent of the layer thickness. For copolymer F - and also copolymers C and D (not shown) - with a hydrophobic block of BUMA, an increase of permeability results even over a long time period. When measured, this leads to a continuous increase of sensitivity if the diffusion layer is applied onto the sensor with distributed enzyme layer. This explains the observed positive sensor drift.

Vice versa, a sensor having block copolymer A, shows a negligible drift, which is due to a very low permeability alteration in the conductivity measurement. Directly after starting measurement (until about 1 h afterwards), however, a strong increase of conductivity is observed in copolymer A. Here, a very fast startup is observed, which is terminated after about 1 hour. At this time, the diffusion layer is completely wetted and has terminated its structural reorganisation due to water uptake. The extent of the structural change presumably depends on the Tg. It seems plausible that a copolymer having an increased Tg passes a reorganisation, which is limited in time and amplitude, as compared to a copolymer having a Tg in the range of the ambient temperature.

In addition, it has to be stated that sensors with copolymer A show a comparatively high sensitivity at the start of measurements as compared to sensors having a copolymer F diffusion layer. This is to be expected due to the identical relative ratios between hydrophobic and hydrophilic blocks. The achieved sensitivity range of 1 to 1.5 nA/mM (see Example 1) is deemed ideal. This sensitivity is likewise obtained for sensors having a diffusion layer consisting of copolymer A.

Regarding the sum of the three physico-chemical characteristics - permeability, mechanic stability and permeability response - an optimal sensor may preferably be obtained with a diffusion layer of a block copolymer, having a hydrophobic block with at least two different randomly arranged hydrophobic monomeric units, such as block copolymer A. None of the other block copolymers, whose hydrophobic blocks only consist of a single monomeric unit reaches a quality, which could be compared in all three parameters with copolymer A.

**Example 4** Characterization of block copolymers

A multiple field sensor (10 fields of working electrodes and counterelectrodes, respectively) for the continuous measurement of the glucose was produced and characterized *in-vitro.*

The sensor was provided with a diffusion layer consisting of a block copolymer comprising a hydrophobic block of random copolymerized methyl methacrylate (MMA) and n-butyl methacrylate (BUMA) and a hydrophilic block of 2-hydroxyethyl methacrylate (HEMA). These polymers (specified G and H) had been produced by Polymer Source, Montreal, and are more permeable than polymer A from Examples 1-3.

In the following Table 2, the copolymers are described:

| Polymer | G | H | A |
|---|---|---|---|
| Molecular weights Mn [kD] | 23.5-b-29 | 21-b-20.5 | 21-b-15 |
| Weight-% HEMA | 55.2 | 49.4 | 41.6 |
| Mol% HEMA (stoichiometrically) | 53.5 | 47.4 | 40 |
| Mol% HEMA (measured by $^{1}H,^{13}C$ NMR) | 51 | 46 | 32.6 |
| Tg [°C] hydrophobic block | 65 | 68 | 86 |
| HEMA monomeric units | 223 | 157 | 115 |
| MMA monomeric units | 194 | 174 | 174 |

The molecular weights Mn of each block are separately indicated in the above Table 2 and represent average values, as polymers are known to have distributions of molecular chain lengths around a specified mean value. This also applies to the derived quantities in Table 2.

The indicated glass transition temperatures of the hydrophobic block are within the desired range in order to guarantee mechanical flexibility.

The decisive parameter with regard to the permeability of the diffusion barrier for the analyte is the sensitivity per area unit of the working electrode (i.e. the geometric area). The sensitivity SE was calculated from current (I) measurements at 10 mM and at 0 mM glucose concentration in phosphate-buffered solution (pH 7.4) in nA/mM:

$$SE = [I(10\ mM) - I(0\ mM)]/10$$

for each of the analyzed sensors. From the individual measurement values (N=8) the mean sensitivity $SE_m$ was determined. The obtained sensitivity values were divided by the microscopically measured geometric total area F of all working electrode spots on the multi-field sensor. Thereby, a sensitivity density $SE_m/F$ was obtained.

The linearity Y of the *in-vitro* function curve is an indication of the diffusion control functionality of the polymer cover layer on the working electrode. It was calculated from current measurements at 20 mM, 10 mM and 0 mM glucose concentration in %:

$$Y^{20mM} = 50 \cdot [I(20mM) - I(0mM)]/[I(10mM) - I(0mM)]$$

for each of the analyzed sensors. From the individual measurement values the mean linearity value and its standard deviation were determined (cf. Table 3).

Finally, the layer thickness L of the diffusion barrier of the sensors was determined by optical measurement for each of the polymers. The corresponding mean values were computed for a sample of $\geq$ 23 sensors with the same polymer. Therefrom, the effective diffusion coefficient $D_{eff}$ of the cover layer may be calculated:

$$D_{eff} = SE_m/F \cdot L_m \cdot 5.182 \cdot 10^{-8}$$

in $cm^2/s$, wherein $SE_m$ and $L_m$ are the respective mean values for the sensitivity and the layer thickness, and F is the total area of all working electrode spots.

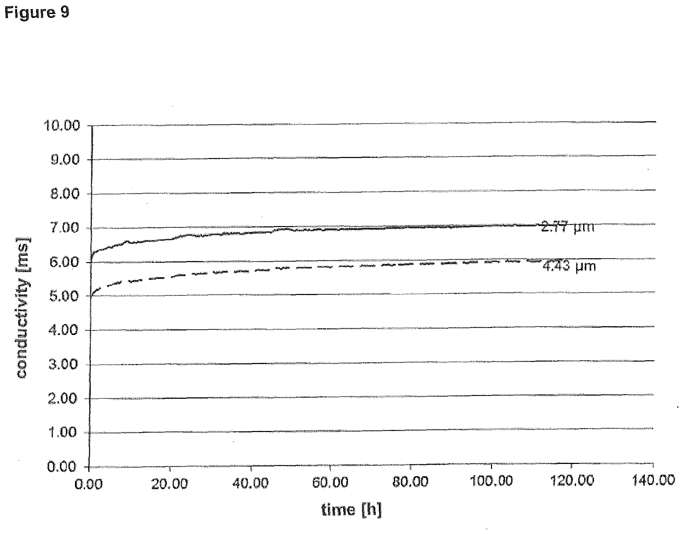

The sensor drift was calculated from repetitions of the glucose concentration stages over 7 days of *in-vitro* measurements. The results for polymer H showing a substantially constant conductivity are depicted in Figure 9.

The following Table 3 shows the results of the functional characterization:

| Polymer | G | H |
|---|---|---|
| $SE_m/F$ [nA/mM*mm$^2$)] | 1.85 | 1.25 |
| Drift [%d] | -1.5$\pm$0.2 | 0.3$\pm$0.1 |
| $Y^{20mM}$[%] | 88.2$\pm$0.7 | 88.6$\pm$0.3 |

(continued)

| Polymer | G | H |
|---|---|---|
| layer thickness $L_m$ [μm] | 11.61 | 12.69 |
| $D_{eff}$ [cm$^2$/s] | $1.11305 \cdot 10^{-9}$ | $8.22019 \cdot 10^{-10}$ |

For the more hydrophilic polymer G (which is more permeable for glucose) the diffusion coefficient was also determined with an alternative method, e.g. permeation of glucose from a chamber with a glucose solution into a chamber with a glucose-free buffer through a film of the polymer. According to this method, a similar value for the diffusion coefficient was obtained ($1.17 \cdot 10^{-9}$ cm$^2$/s).

**Example 5** Protein binding to spacer layer material

To assess protein binding to spacer layer materials, ethanolic solutions of Adapt® (Biointeractions Ltd, Reading, England) or Eudragit E100 (Evonik Industries) were filled into an incubation plate (FluoroNunc Maxisorp, Thermo Scientific). Eudragit E100 is a cationic copolymer based on dimethylaminoethyl methacrylate, butyl methacrylate and methyl methacrylate. The polymers were dried over night at 40°C. Thereafter the spacer materials were overlaid with fibrinogen solution. The solution contained fibrinogen from human plasma that was conjugated to the fluorescent dye Alexa488 (purchased from Invitrogen). After 4 h incubation the fibrinogen solution was aspirated and the spacer layers were washed eight times with borate buffer. The amount of spacer-bound protein was analysed by measuring the fluorescence intensity in the incubation plate at an excitation wavelength of 485 nm and an emission of 528 nm using a fluorescence reader (Synergy4, BioTek Instruments). Known concentrations of labelled protein (6.25 - 500 ng) were used to prepare a calibration curve to convert fluorescence readings to amount of protein.

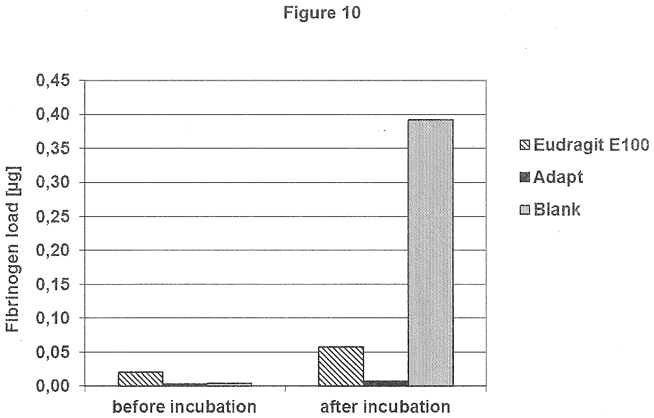

As expected, fibrinogen attached to the uncoated incubation plate (Blank) resulting in 390 ng of bound protein (Fig.10). The plate coated with Eudragit E100 showed reduced protein binding of 60 ng. Hardly any protein binding was detectable in the Adapt® coated plate. The readings before incubation resulted from background fluorescence. These results clearly demonstrate that surfaces coated with the spacer materials, especially those coated with Adapt®, are well protected against fibrinogen adhesion.

**Example 6** Cytokine release by cells after contact with spacer layer

Sensors were manufactured as described in Example 2. Afterwards, the sensors were provided with a spacer layer as described in Example 2. The spacer layer was made of Lipidure CM 5206 (NOF Corporation, Japan) or was made of Adapt® (Biointeractions Ltd, Reading, England).

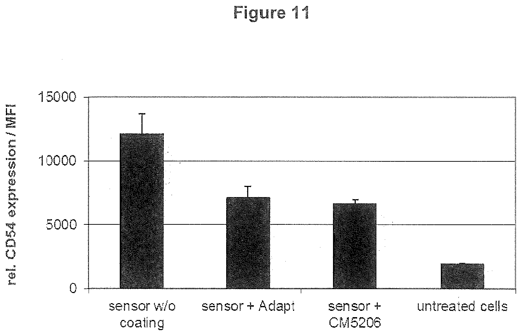

Sensors without spacer layer, sensors with spacer layer made of Lipidure CM 5206 or sensors with spacer layer made of Adapt® were incubated with monocytic THP-1 cells and induction of inflammatory markers was analysed. THP-1 cells were cultured in the presence of the sensors for 24 h at 37°C. The cells were then collected by centrifugation. The supernatant was used to determine the release of cytokines whereas the cell pellet was resuspended in PBS containing 1 % of bovine serum albumin (BSA) and used to analyse expression of the cell surface protein CD54 (also known as ICAM-1, an inflammatory biomarker). THP-1 cells were incubated with an anti-CD54 antibody conjugated with the fluorescent dye phycoerythrin (BD Bioscience). After incubation for 45 min at 4°C, cells were washed in PBS/1 % BSA and the mean fluorescence intensity (MFI) of 10000 cells was determined using a flow cytometer (excitation wavelength 532 nm, emission wavelength 585 nm) (BD FACSArray, BD Bioscience). Compared to untreated THP-1 cells, incubation with sensors without coating resulted in increased relative CD54 expression (6-fold induction) as indicated by high MFI readings (Fig. 11). Incubating cells with sensors covered with spacer layers of CM 5206 or Adapt® resulted in attenuation of CD54 expression by 45 % or 41 % respectively.

The supernatant was used to determine the amount of the cytokines Interleukin- 8 (IL-8) and "monocyte chemotactic protein-1" (MCP-1) using a bead-based immunoassay according to the manufacturer's instructions (Flex sets, BD Bioscience) and subsequent flow cytometric analysis (BD FACSArray, BD Bioscience). Data analysis was performed using FCAP array software v1.0.1 (Soft flow Hungary Ltd.).

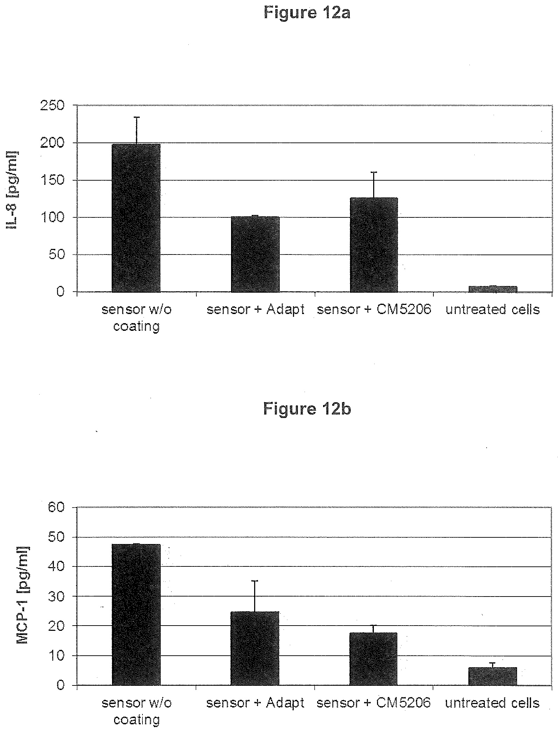

Compared to untreated THP-1 cells sensors without coating induced a strong release of IL-8 (49 vs. 197 pg/ml) (Fig. 12a) and MCP-1(6 vs.48 pg/ml) (Fig. 12b). The release of IL-8 and MCP-1 was reduced when sensors were covered with spacer layers of CM 5206 or Adapt® Sensors covered with Adapt® induced the release of 100 pg/ml of IL-8 and 25 pg/ml of MCP-1. Sensors covered with CM 5206 resulted in secretion of 125 pg/ml of IL-8 and 18 pg/ml of MCP-1.

Collectively, these data indicate that the spacer layers attenuate the induction of three well known inflammation biomarkers, namely CD54, IL-8 and MCP-1.

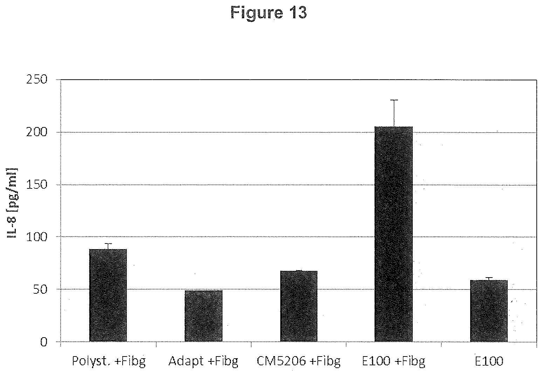

To analyse the role of protein adsorption for activation of THP-1 cells, tissue culture plates were coated with CM 5206, Adapt® or Eudragit E100. The spacer was then incubated with human fibrinogen (Sigma-Aldrich). THP-1 cells were incubated with different spacers + fibrinogen layers. After 48 h incubation at 37°C the cells were sedimented by centrifugation and the supernatant was analysed for IL-8 release. As a control, cells were grown in culture plates without spacer but coated with fibrinogen (Polyst. = culture plate material). As shown in Fig. 13, these cells released 89 pg/ml of IL-8. Cells cultured on CM 5206 + fibrinogen or on Adapt® + fibrinogen released 68 or 49 pg/ml, respectively. In contrast, cells grown on Eudragit E100 + fibrinogen released 206 pg/ml of IL-8. Notably, cells grown on Eudragit E100 without fibrinogen-coating secreted only 59 pg/ml of IL-8. Adsorption of fibrinogen on the polymer surface and conformational changes in the protein might expose the MAC-1 binding site. THP-1 cells that are activated via binding to their MAC-1 receptor release cytokines, like IL-8, and thereby trigger an inflammatory response. Hence, spacer layers made of Adapt® or CM 5206 avoid protein deposition and exposure of structural motifs on surfaces (like sensors) and thereby minimise inflammatory reactions against implants.

**Example 7** <u>Limited haemolysis of sensors coated with a spacer layer</u>

Sensors were manufactured as described in Example 2. Afterwards, the sensors were provided with a spacer layer as described in Example 2. The spacer layer was made of Lipidure CM 5206 (NOF Corporation, Japan) or was made of Adapt® (Biointeractions Ltd, Reading, England).

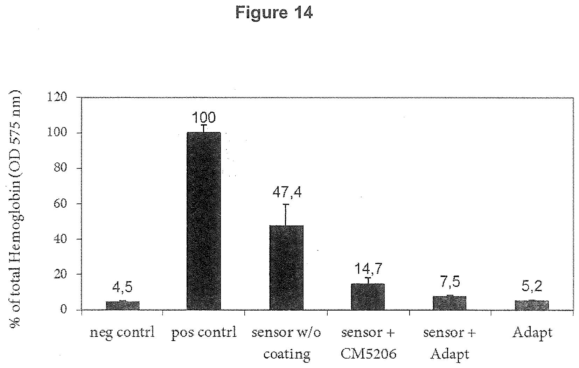

The haemolytic potential of sensors without spacer layer, sensors with spacer layer made of Lipidure CM 5206 or sensors with spacer layer made of Adapt® was analysed. Therefore, sensors with a total surface area of 6 cm$^2$ were incubated with red blood cells and then the lysis was determined by measuring the release of haemoglobin to the supernatant. Erythrocytes were isolated from fresh human blood by centrifugation (citrate was used to avoid coagulation). They were then washed with phosphate buffered saline (PBS) and thereafter diluted 1:40 in PBS. The erythrocyte suspension was incubated with the sensors for 24 h at 37°C in the dark on a rotation platform (350 rpm). Afterwards, the cells were sedimented by centrifugation and the haemoglobin content of the supernatant was determined spectroscopically by measuring the absorption of the supernatant at a wavelength of 575 nm. The results are presented as lytic index in %, which is the release of haemoglobin in a sample divided by haemoglobin release in the positive control (= complete osmotic lysis of erythrocytes in distilled water). The results are shown in Fig. 14.

Sensors without a spacer layer significantly caused haemolysis as indicated by a high haemolytic index of 47.4 %. Coating the sensors with a spacer layer of Lipidure CM 5206 reduced the haemolytic potential of the sensors as demonstrated by a lytic index of 14.7 %. Sensors coated with a spacer layer of Adapt® marginally caused haemolysis resulting in a lytic index of 7.5 % which is in the range of the negative control (= erythrocytes in PBS incubated without any test material) or Adapt® alone. These results indicated the protective function of the spacer layer to reduce haemolysis.

**Claims**

1. An electrode system for measuring the concentration of an analyte under *in-vivo* conditions, comprising an electrode with immobilized enzyme molecules, optionally a diffusion barrier that controls diffusion of the analyte from the exterior of the electrode system to the enzyme molecules,
   **characterised in that** a spacer membrane forms at least a portion of the outer layer of the electrode system and wherein the spacer membrane comprises a hydrophilic copolymer of acrylic and/or methacrylic monomers, wherein the hydrophilic copolymer comprises more than 50 mol-% hydrophilic monomers.

2. The electrode system of claim 1, wherein the spacer membrane is a hydrophilic copolymer from at least 2 or 3 acrylic and/or methacrylic monomers.

3. The electrode system of claim 1 or 2, wherein the hydrophilic monomers are selected from hydrophilic (meth)acrylesters with a polar, e.g. OH, $OCH_3$ or $OC_2H_2$ group, hydrophilic (meth)acrylamides, (meth)acrylic acid or combinations thereof, preferably selected from:

   2-hydroxyethyl acrylate,
   2-hydroxyethyl methacrylate (HEMA),
   2-methoxyethyl acrylate,
   2-methoxyethyl methacrylate,
   2-ethoxyethyl acrylate,
   2-ethoxyethyl methacrylate,

2- or 3-hydroxypropyl acrylate,
2- or 3-hydroxypropyl methacrylate (2- or 3-HPMA),
2- or 3-methoxypropyl acrylate,
2- or 3-methoxypropyl methacrylate,
2- or 3-ethoxypropyl acrylate,
2- or 3-ethoxypropyl methacrylate,
1- or 2-glycerol acrylate,
1- or 2-glycerol methacrylate,
acrylamide,
methacrylamide,
an N-alkyl- or N,N-dialkyl acrylamide, and
an N-alkyl- or N,N-dialkyl methacrylamide,
wherein alkyl comprises 1-3 C-atoms,
acrylic acid,
methacrylic acid
and combinations thereof,

more preferably from 2-hydroxyethyl methacrylate (HEMA), 2-hydroxypropyl methacrylate (2-HPMA) and combinations thereof.

4. The electrode system of any one of claims 1-3, wherein the hydrophilic copolymer of the spacer membrane comprises at least 60 mol-% or at least 70 mol-% hydrophilic monomers.

5. The electrode system of any one of claims 1-4, wherein the copolymer of the spacer membrane further comprises up to 40 mol-% or up to 30 mol-% hydrophobic monomers, preferably selected from:

methyl acrylate,
methyl methacrylate (MMA),
ethyl acrylate,
ethyl methacrylate (EMA),
n- or i-propyl acrylate,
n- or i-propyl methacrylate,
n-butyl acrylate,
n-butyl methacrylate (BUMA),
neopentyl acrylate,
neopentyl methacrylate,
and combinations thereof,

more preferably from methyl methacrylate (MMA), n-butyl methacrylate (BUMA) and combinations thereof.

6. The electrode system of claim 5, wherein the hydrophobic monomers are methyl methacrylate (MMA) or n-butyl methacrylate (BUMA) and the hydrophilic monomers are 2-hydroxyethyl methacrylate (HEMA) and/or 2-hydroxypropyl methacrylate (2-HPMA).

7. The electrode system of claim 5 or 6, wherein the spacer membrane comprises a copolymer from n-butyl methacrylate (BUMA), 2-hydroxyethyl methacrylate (2-HEMA) and 2-hydroxypropyl methacrylate (2-HPMA), wherein the copolymer comprises more than 80 mol-% 2-HEMA monomers.

8. The electrode system of any one of claims 1-7, wherein the hydrophilic copolymer is a random copolymer or a block copolymer.

9. The electrode system of any one of claims 1-8, wherein the thickness of the spacer membrane is less than about 20 $\mu$m, preferably less than 3 $\mu$m, more preferably from about 1 to about 3 $\mu$m.

10. The electrode system of any one of claims 1-9, wherein the relative water uptake of the hydrophilic copolymer does not exceed 50 weight-%, preferably 40 wt-%, more preferably 30 wt-%, based on the total weight of the copolymer.

11. The electrode system of any one of claims 1-10, wherein the diffusion barrier comprises a hydrophilic polyurethane or a block copolymer having at least one hydrophilic block and at least one hydrophobic block.

12. The electrode system of any one of claims 1-11, comprising a counterelectrode (2) having an electrical conductor (2a), a working electrode (1) having an electric conductor (1a) on which the immobilized enzyme molecules (5) and optionally the diffusion barrier (8) are arranged and a spacer membrane (9), which covers at least the working electrode (1) and optionally also the counterelectrode (2).

13. A sensor which is insertable or implantable into a body comprising an electrode system of any one of claims 1-12, wherein the sensor is preferably for the measurement of glucose.

14. Use of a hydrophilic copolymer of acrylic and/or methacrylic monomers, wherein the hydrophilic copolymer comprises more than 50 mol-% hydrophilic monomers as a spacer membrane for an enzymatic electrode system.

15. The use of claim 14, for minimizing the foreign body reaction (FRB) against the enzymatic electrode system.


**Patentansprüche**

1. Elektrodensystem zur Messung der Konzentration eines Analyten unter in vivo-Bedingungen, umfassend eine Elektrode mit immobilisierten Enzymmolekülen, gegebenenfalls eine Diffusionsbarierre, die die Diffusion des Analyten von außerhalb des Elektrodensystems zu den Enzymmolekülen kontrolliert,
**dadurch gekennzeichnet, dass** eine Spacermembran mindestens einen Teil der äußeren Schicht des Elektrodensystems bildet und worin die Spacermembran ein hydrophiles Copolymer aus Acryl- und/oder Methacrylmonomeren umfasst, wobei das hydrophile Copolymer mehr als 50 Mol-% hydrophile Monomere umfasst.

2. Elektrodensystem nach Anspruch 1, wobei die Spacermembran ein hydrophiles Copolymer aus mindestens 2 oder 3 Acryl- und/oder Methacrylmonomeren ist.

3. Elektrodensystem nach Anspruch 1 oder 2, wobei die hydrophilen Monomere ausgewählt sind aus hydrophilen (Meth)acrylestern mit einer polaren, z.B. OH-, $OCH_3$- oder $OC_2H_5$-Gruppe, hydrophilen (Meth)acrylamiden, (Meth)acrylsäure oder Kombinationen davon, vorzugsweise ausgewählt aus:

2-Hydroxyethylacrylat,
2-Hydroxyethylmethacrylat (HEMA),
2-Methoxyethylacrylat,
2-Methoxyethylmethacrylat,
2-Ethoxyethylacrylat,
2-Ethoxyethylmethacrylat,
2- oder 3-Hydroxypropylacrylat,
2- oder 3-Hydroxypropylmethacrylat (2- oder 3-HPMA),
2- oder 3-Methoxypropylacrylat,
2- oder 3-Methoxypropylmethacrylat,
2- oder 3-Ethoxypropylacrylat,
2- oder 3-Ethoxypropylmethacrylat,
1- oder 2-Glycerolacrylat,
1- oder 2-Glycerolmethacrylat,
Acrylamid,
Methacrylamid,
einem N-alkyl- oder N,N-dialkylacrylamid und
einem N-alkyl- oder N,N-dialkylmethacrylamid,
worin Alkyl 1-3 C-Atome umfasst,
Acrylsäure,
Methacrylsäure
und Kombinationen davon,

stärker bevorzugt aus 2-Hydroxyethylmethacrylat (HEMA), 2-Hydroxypropylmethacrylat (2-HPMA) und Kombinationen davon.

**4.** Elektrodensystem nach einem der Ansprüche 1-3, wobei das hydrophile Copolymer der Spacermembran mindestens 60 Mol-% oder mindestens 70 Mol-% hydrophile Monomere umfasst.

**5.** Elektrodensystem nach einem der Ansprüche 1-4, wobei das Copolymer der Spacermembran ferner bis zu 40 Mol-% oder bis zu 30 Mol-% hydrophobe Monomere umfasst, vorzugsweise ausgewählt aus

Methylacrylat,
Methylmethacrylat (MMA),
Ethylacrylat,
Ethylmethacrylat (EMA),
n- oder i-Propylacrylat,
n- oder i-Propylmethacrylat,
n-Butylacrylat,
n-Butylmethacrylat (BUMA),
Neopentylacrylat,
Neopentylmethacrylat,
und Kombinationen davon,

stärker bevorzugt aus Methylmethacrylat (MMA), n-Butylmethacrylat (BUMA) und Kombinationen davon.

**6.** Elektrodensystem nach Anspruch 5, wobei die hydrophoben Monomere Methylmethacrylat (MMA) oder n-Butylmethacrylat (BUMA) sind und die hydrophilen Monomere 2-Hydroxyethylmethacrylat (HEMA) und/oder 2-Hydroxypropylmethacrylat (2-HPMA) sind.

**7.** Elektrodensystem nach Anspruch 5 oder 6, wobei die Spacermembran ein Copolymer aus n-Butylmethacrylat (BUMA), 2-Hydroxyethylmethacrylat (2-HEMA) und 2-Hydroxypropylmethacrylat (2-HPMA) umfasst, wobei das Copolymer ca. 80 Mol-% an 2-HEMA-Monomeren umfasst.

**8.** Elektrodensystem nach einem der Ansprüche 1-7, wobei das hydrophile Copolymer ein Random-Copolymer oder ein Block-Copolymer ist.

**9.** Elektrodensystem nach einem der Ansprüche 1-8, wobei die Dicke der Spacermembran geringer als ca. 20 $\mu$m, bevorzugt geringer als 5 $\mu$m, stärker bevorzugt von ca. 1 bis ca. 3 $\mu$m ist.

**10.** Elektrodensystem nach einem der Ansprüche 1-9, wobei die relative Wasseraufnahme des hydrophilen Copolymers 50 Gew.-%, bevorzugt 40 Gew.-%, stärker bevorzugt 30 Gew.-%, basierend auf dem Gesamtgewicht des Copolymers, nicht übersteigt.

**11.** Elektrodensystem nach einem der Ansprüche 1-10, wobei die Diffusionsbarierre ein hydrophiles Polyurethan oder ein Block-Copolymer, welches mindestens einen hydrophilen Block und mindestens einen hydrophoben Block aufweist, umfasst.

**12.** Elektrodensystem nach einem der Ansprüche 1-11, umfassend eine Gegenelektrode (2), die einen elektrischen Leiter (2a) aufweist, eine Arbeitselektrode (1), die einen elektrischen Leiter (1a) aufweist, auf welchem die immobilisierten Enzymmoleküle (5) und gegebenenfalls die Diffusionsbarierre (8) angeordnet sind, und eine Spacermembran (9), die mindestens die Arbeitselektrode (1) und gegebenenfalls auch die Gegenelektrode (2) bedeckt.

**13.** Ein Sensor, der in einen Körper einführbar oder implantierbar ist, umfassend ein Elektrodensystem nach einem der Ansprüche 1-12, wobei der Sensor vorzugsweise zur Messung von Glucose ist.

**14.** Verwendung eines hydrophilen Copolymers aus Acryl- und/oder Methacrylmonomeren, wobei das hydrophile Copolymer mehr als 50 Mol-% hydrophile Monomere umfasst, als eine Spacermembran für ein enzymatisches Elektrodensystem.

**15.** Verwendung nach Anspruch 14 zur Minimierung der Fremdkörperreaktion (*foreign body reaction*, FRB) gegen das enzymatische Elektrodensystem.

**Revendications**

1. Système d'électrodes pour mesurer la concentration d'un analyte dans des conditions *in vivo*, comprenant une électrode avec des molécules d'enzyme immobilisées, éventuellement une barrière de diffusion qui contrôle la diffusion de l'analyte depuis l'extérieur du système d'électrodes vers les molécules d'enzyme, **caractérisé en ce qu'**une membrane d'espacement forme au moins une partie de la couche externe du système d'électrodes et dans lequel la membrane d'espacement comprend un copolymère hydrophile de monomères acryliques et/ou méthacryliques, dans lequel le copolymère hydrophile comprend plus de 50 % en moles de monomères hydrophiles.

2. Système d'électrodes selon la revendication 1, dans lequel la membrane d'espacement est un copolymère hydrophile issu d'au moins 2 ou 3 monomères acryliques et/ou méthacryliques.

3. Système d'électrodes selon la revendication 1 ou 2, dans lequel les monomères hydrophiles sont choisis parmi les (méth)acrylesters hydrophiles avec un groupe polaire, par exemple, OH, $OCH_3$ ou $OC_2H_5$, les (méth)acrylamides hydrophiles, l'acide (méth)acrylique ou les combinaisons de ceux-ci, choisis de préférence parmi :

   l'acrylate de 2-hydroxyéthyle,
   le méthacrylate de 2-hydroxyéthyle (HEMA),
   l'acrylate de 2-méthoxyéthyle,
   le méthacrylate de 2-méthoxyéthyle,
   l'acrylate de 2-éthoxyéthyle,
   le méthacrylate de 2-éthoxyéthyle,
   l'acrylate de 2- ou 3-hydroxypropyle,
   le méthacrylate de 2- ou 3-hydroxypropyle (2- ou 3-HPMA),
   l'acrylate de 2 ou 3-méthoxypropyle,
   le méthacrylate de 2- ou 3-méthoxypropyle,
   l'acrylate de 2- ou 3-éthoxypropyle
   le méthacrylate de 2- ou 3-éthoxypropyle,
   l'acrylate de 1- ou 2-glycérol,
   le méthacrylate de 1- ou 2-glycérol,
   l'acrylamide,
   le méthacrylamide,
   un N-alkyl- ou N,N-dialkyl acrylamide, et
   un N-alkyl- ou N,N-dialkylméthacrylamide,
   dans lesquels l'alkyle comprend 1 à 3 atomes de carbone,
   l'acide acrylique
   l'acide méthacrylique
   et les combinaisons de ceux-ci,

   plus préférablement parmi le méthacrylate de 2-hydroxyéthyle (HEMA), le méthacrylate de 2-hydroxypropyle (2-HPMA) et les combinaisons de ceux-ci.

4. Système d'électrodes selon l'une quelconque des revendications 1 à 3, dans lequel le copolymère hydrophile de la membrane d'espacement comprend au moins 60 % en moles ou au moins 70 % en moles de monomères hydrophiles.

5. Système d'électrodes selon l'une quelconque des revendications 1 à 4, dans lequel le copolymère de la membrane d'espacement comprend en outre jusqu'à 40 % en moles ou jusqu'à 30 % en mole de monomères hydrophobes, choisi de préférence parmi :

   l'acrylate de méthyle,
   le méthacrylate de méthyle (MMA),
   l'acrylate d'éthyle,
   le méthacrylate d'éthyle (EMA),
   l'acrylate de n- ou i-propyle,
   le méthacrylate de n- ou i-propyle,
   l'acrylate de n-butyle,

le méthacrylate de n-butyle (BUMA),
l'acrylate de néopentyle,
le méthacrylate de néopentyle,
et les combinaisons de ceux-ci,

plus préférablement parmi le méthacrylate de méthyle (MMA), le méthacrylate de n-butyle (BUMA) et les combinaisons de ceux-ci.

6. Système d'électrodes selon la revendication 5, dans lequel les monomères hydrophobes sont le méthacrylate de méthyle (MMA) ou le méthacrylate de n-butyle (BUMA) et les monomères hydrophiles sont le méthacrylate de 2-hydroxyéthyle (HEMA) et/ou le méthacrylate de 2-hydroxypropyle (2-HPMA).

7. Système d'électrodes selon la revendication 5 ou 6, dans lequel la membrane d'espacement comprend un copolymère à partir de méthacrylate de n-butyle (BUMA), de méthacrylate de 2-hydroxyéthyle (2-HEMA) et de méthacrylate de 2-hydroxypropyle (2-HPMA), dans lequel le copolymère comprend environ 80 % en moles de monomères 2-HEMA.

8. Système d'électrodes selon l'une quelconque des revendications 1 à 7, dans lequel le copolymère hydrophile est un copolymère aléatoire ou un copolymère séquencé.

9. Système d'électrodes selon l'une quelconque des revendications 1 à 8, dans lequel l'épaisseur de la membrane d'espacement est inférieure à environ 20 $\mu$m, de préférence inférieure à 5 $\mu$m, plus préférablement d'environ 1 à environ 3 $\mu$m.

10. Système d'électrodes selon l'une quelconque des revendications 1 à 9, dans lequel l'absorption relative d'eau du copolymère hydrophile ne dépasse pas 50 % en poids, de préférence 40 % en poids, plus préférablement 30 % en poids, par rapport au poids total du copolymère.

11. Système d'électrodes selon l'une quelconque des revendications 1 à 10, dans lequel la barrière de diffusion comprend un polyuréthanne hydrophile ou un copolymère séquencé ayant au moins un bloc hydrophile et au moins un bloc hydrophobe.

12. Système d'électrodes selon l'une quelconque des revendications 1 à 11, comprenant une contre-électrode (2) ayant un conducteur électrique (2a), une électrode de travail (1) ayant un conducteur électrique (1a) sur lequel les molécules d'enzyme immobilisées (5) et éventuellement la barrière de diffusion (8) sont disposées, et une membrane d'espacement (9), qui recouvre au moins l'électrode de travail (1) et éventuellement aussi la contre-électrode (2).

13. Capteur qui peut être inséré ou implanté dans un corps comprenant un système d'électrodes selon l'une quelconque des revendications 1 à 12, dans lequel le capteur est de préférence destiné à la mesure du glucose.

14. Utilisation d'un copolymère hydrophile de monomères acryliques et/ou méthacryliques, dans laquelle le copolymère hydrophile comprend plus de 50 % en moles de monomères hydrophiles en tant que membrane d'espacement pour un système d'électrodes enzymatique.

15. Utilisation selon la revendication 14, pour minimiser la réaction de corps étrangers (FRB) contre le système d'électrodes enzymatique.

# Figures 1-3

Fig. 1

Fig. 2

Fig. 3

Figure 4

**Figure 5**

Figure 6

Figure 7

Time [h]

Figure 8

Figure 9

Figure 10

Figure 11

Figure 12a

Figure 12b

Figure 13

Figure 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007147475 A
- WO 2010028708 A
- WO 2006058779 A
- EP 2163190 A

**Non-patent literature cited in the description**

- **BÖKER et al.** *Macromolecules,* 2001, vol. 34, 7477-7488